# EUROPÄISCHE PATENTSCHRIFT

(11) **EP 0 817 675 B1**
(45) Veröffentlichungstag und Bekanntmachung des Hinweises auf die Patenterteilung: **02.06.1999**
(21) Anmeldenummer: 96908140.5
(22) Anmeldetag: 26.03.1996
(51) Int. Cl.: B01J 21/16, C10G 29/16, C07C 7/148

(54) **KATALYSATOR ZUR ENTFERNUNG VON OLEFINEN AUS AROMATEN ODER AROMATENGEMISCHEN**
CATALYST FOR REMOVING OLEFINS FROM AROMATIC COMPOUNDS OR MIXTURES THEREOF
CATALYSEUR SERVANT A ELIMINER LES OLEFINES CONTENUES DANS DES COMPOSES AROMATIQUES OU DES MELANGES DE COMPOSES AROMATIQUES

(30) Priorität: 31.03.1995 DE 19512134
(43) Veröffentlichungstag der Anmeldung: 14.01.1998
(73) Patentinhaber: SÜD-CHEMIE AG, D-80333 München (DE)
(72) Erfinder: HÄHN, Reinhard, D-84186 Vilsheim (DE); ENGELHARDT, Thomas, D-85356 Freising (DE); FLESSNER, Uwe, D-81375 München (DE); ZSCHAU, Werner, D-82237 Steinebach (DE)
(74) Vertreter: Reitzner, Bruno, Dr.
(86) Internationale Anmeldenummer: EP9601320
(87) Internationale Veröffentlichungsnummer: WO9630119

(56) Entgegenhaltungen:
- EP-A- 0 116 469
- EP-A- 0 449 453
- DE-B- 1 094 389
- FR-A- 2 385 789
- FR-A- 2 599 275
- US-A- 3 835 037

## Beschreibung

Die Erfindung betrifft die Verwendung eines Katalysators auf der Basis eines smektitischen Tonminerals zur Entfernung von Olefinen aus Aromaten oder Aromatengemischen.

Die industriell bedeutendsten Aromaten Benzol, Toluol und die Xylole (BTX) werden heute nahezu ausschließlich durch katalytische oder thermische Umwandlung geeigneter Erdölfraktionen hergestellt.

Beim sogenannten katalytischen Reformieren wird eine paraffinische Naphthafraktion bei etwas 400° C mit Edelmetallbeschichteten Katalysatoren behandelt. Wahrend dieses katalytischen Prozesses werden aus den gesättigten Kohlenwasserstoffen Aromaten gebildet. Diese Aromaten werden dann durch eine Extraktion bzw. Kristallisation von den Nichtaromaten abgetrennt und destillativ weiterverarbeitet.

Als Wichtigstes Verfahren zur extraktiven Abtrennung der Benzol-Toluol-Mischungen hat sich das Sulfolan-Verfahren durchgesetzt (Ullmanns Enzyklopädie der technischen Chemie, Bd.8(1974),S.395).

Neben den gewünschten Aromaten werden während des katalytischen Reformierung auch geringe Mengen an Olefinen gebildet. Diese Olefine, deren Gehalt in der Regel unter 1 % liegt, stören den Weiterverarbeitungsprozeß und müssen entfernt werden. Da die unerwünschten Olefine etwa die gleichen Siedepunkte aufweisen wie die Aromaten, ist eine destillative Abtrennung nicht möglich.

Als wirtschaftliches Verfahren zur Entfernung dieser Olefine hat sich weltweit die katalytische Behandlung mit Erdalkali-Aluminiumsilicaten, z.B. aktivierten Smektiten in Granulatform, durchgesetzt. Hierbei wird der Aromatenstrom bei etwa 150 - 200° C durch einen Festbettreaktor geleitet. Die Granulate wirken hierbei als Katalysator; die unerwünschten Olefine werden in höhersiedende Produkte umgewandelt, die dann destillativ leicht abgetrennt werden können.

Es ist bekannt, daß als geeignete Katalysatoren zur Entfernung der Olefine bevorzugt natürliche oder synthetische ErdalkaliAluminiumsilicate eingesetzt werden. So sind die unterschiedlichsten Verfahren unter Verwendung von säureaktivierten Bentoniten (Bleicherden) beschrieben. In diesem Zusammenhang sei beispielsweise auf GB-1 162 945 und die DE-C-22 36 996 verwiesen. Bei den kommerziellen Produkten für die Aromatenreinigung handelt es sich in der Regel um granulierte säureaktivierte Bentonite, wie sie zur Raffination von Nahrungsmittelölen eingesetzt werden. Die Produkte werden in der Regel in einem Korngrößenbereich zwischen 0,3 und 0,6 mm angeboten, die spezifische Oberfläche schwankt zwischen 200 und 400 m², die Ionenumtauschfähigkeit (IUF) zwischen 30 und 60 mVal/100g.

Neben den bevorzugt verwendeten säureaktivierten Bentoniten können auch synthetische Silicate, wie Al-Silicate, Mg-Silicate, Zr-Silicate, eingesetzt werden.

In der US-A 4 795 550 ist die Verwendung von Zeolithen zur Entfernung von Olefinen aus Aromatenfraktionen beschrieben. Zeolithe sind zwar sehr reaktiv; es kommt aber in ihrem engen Porensystem zur Bildung von polymeren Nebenprodukten, die zu einer sehr schnellen Desaktivierung des Katalysatorbettes führen.

Die Standzeit der säureaktivierten Bentonite in Festbett-Reaktoren schwankt je nach Prozeßbedingungen sehr stark und liegt zwischen einigen Wochen und einem Jahr. Danach ist durch Desaktivierungsprozesse so viel katalytische Aktivität verloren gegangen, daß ein Austausch des Katalysators notwendig wird. Die Prozeßbetreiber derartiger Aromatenanlagen sind daher an einem hochaktiven Katalysator interessiert, welcher sich durch eine lange Standzeit auszeichnet.

Die EP-A-449453 beschreibt ein Verfahren zur Herstellung von Oligomeren oder Co-Oligomeren aus linearen Olefinen durch Umsetzung mit Katalysatoren auf Smektitbasis, wobei diese Katalysatoren säureaktiviert sind und durch Ionenaustausch mit Lewis-Säuren (Al³⁺-Kationen) modifiziert sein können. Diese Katalysatoren werden nicht zur Entfernung von Olefinen aus Aromaten oder Aromatengemische verwendet.

Aufgabe der vorliegenden Erfindung war es, Katalysatoren zur Entfernung von Olefinen aus Aromaten oder Aromatengemischen auf der Basis von Smektiten mit konstant hoher katalytischer Aktivität und somit langer Standzeit zu entwickeln.

Gegenstand der Erfindung ist somit die Verwendung eines Katalysators auf der Basis eines säureaktivierten smektitischen Tonminerals, welches mindestens 5 mVal/100 g austauschfähige Al³⁺-Kationen enthält, zur Entfernung von Olefinen aus Aromanten oder Aromatengemischen.

Als Ausgangsmaterial für das säurekativierte smektitische Tonmineral wird in erster Linie Bentontit verwendet, dessen Hauptmineral der Montmorillonit ist. Es können aber auch andere smektitische Tonminerale, wie Beidellit, Saponit, Glauconit, Nontronit und Hectorit verwendet werden.

Der erfindungsgemäß verwendete Katalysator ist vorzugsweise dadurch gekennzeichnet, daß das säureaktivierte smektitische Tonmineral die nachstehend angegebenen Eigenschaften hat:
(a) eine spezifische Oberfläche von etwa 50 bis 500 m²/g;
(b) eine Ionenumtauschfähigkeit (IUF) für austauschfähige Kationen von insgesamt etwa 20 bis 60 mVal/100 g, wobei der Anteil der Al³⁺-Ionen an der IUF etwa 10 bis 60 mVal/100 g, der Anteil der Alkali-Ionen an der IUF weniger als etwa 7,0 mVal/100 g und der Anteil der Erdalkali-Ionen an der IUF weniger als etwa 50 mVal/100 g beträgt;
(c) eine Gesamtacidität von mehr als etwa 10 mg KOH/g;
(d) einen Anteil an freien Erdalkali- oder Alkali-Ionen von weniger als 5 mVal/100g;
(e) einen Gehalt an freier Kieselsäure von etwa 15 bis 55 Gew.%.

Vorzugsweise beträgt der Gehalt an austauschfähigen Al³⁺-Ionen etwa 10 bis 50 mVal/100g.

Unter "austauschfähigen Al³⁺-Ionen" versteht man Al³⁺-Ionen, die an Zwischenschichtplätzen und nicht innerhalb der Schichten des smektitischen Tonminerals gebunden sind. Sie sind im Sinne eines Ionenaustauschs chemisch gebunden, so daß sie nicht mit Wasser ausgewaschen werden können. Der Gehalt an austauschfähigen Al³⁺-Ionen wird im Rahmen der Bestimmung der Gesamt-Ionenumtauschfähigkeit (IUF) festgestellt. Hierbei wird eine Probe des Katalysators (2 g) eine Stunde in 100 ml einer 2-normalen NH₄Cl-Lösung unter Rückfluß gekocht und weitere 16 Stunden bei Raumtemperatur stehengelassen. Dann wird die Probe abfiltriert und chloridfrei gewaschen. Zunächst wird die Gesamt-IUF durch Bestimmung der in das Gitter eingetauschten NH₄⁺-Ionen bestimmt. Zu diesem Zweck wird der Gehalt an Ammoniumionen im NH₄⁺-ausgetauschten Katalysator nach Kjeldahl bestimmt. Der Gehalt an austauschfähigen Al³⁺-Ionen wird im Filtrat der NH₄Cl-Kochlösung spektrophotometrisch bestimmt und in mVal je 100 g Katalysator ausgedrückt. Auf die gleiche Weise werden die Gehalte an Alkali- und Erdalkaliionen im Filtrat der NH₄Cl-Kochlösung bestimmt.

Nach der gleichen Methode werden die Gesamt-IUF und der Anteil der austauschfähigen Kationen des säurekativierten smektitischen Ausgangsmaterials bestimmt.

Der erfindungsgemäß verwendete Katalysator kann zusätzlich zu den austauschfähigen Al³⁺-Ionen freie Al³⁺-Ionen auf der Oberfläche, in den Poren und im Zwischenkornvolumen enthalten. Diese Al³⁺-Ionen können durch einfaches Auswaschen des Katalysators mit Wasser entfernt werden.

Zu diesem Zweck wird eine Probe des Katalysators (10 g) vor dem Kochen mit der NH₄Cl-Lösung 5-mal mit je 100 ml Wasser von 25°C mehrmals in einer Filternutsche gewaschen, bis im letzten Waschwasseranteil kein Aluminium mehr nachzuweisen ist. Das Aluminium wird in den vereinigten Waschwässern wiederum spektrophotometrisch bestimmt. Auf die gleiche Weise können auch die anderen freien Kationen bestimmt werden.

Die Bestimmung der IUF und des Anteils der einzelnen Ionen an der Gesamt-IUF sowie die Bestimmung der freien Kationen kann auch bei den nicht säurebehandelten smektitischen Ausgangsmaterialien vorgenommen werden. Hierbei wird durch das Ergebnis ein zu hoher Gehalt an austauschfähigen Calcium oder Magnesiumionen vorgetäuscht. Dies ist dadurch bedingt, daß das smektitische Ausgangsmaterial häufig mit Calcium- und/oder Magnesiumcarbonat verunreinigt ist, die beim Kochen mit der NH₄Cl-Lösung gelöst werden.

Man nimmt an, daß sowohl die austauschfähigen Al³⁺-Ionen als auch die freien Al³⁺-Ionen eine katalytische Wirkung nach Art der Lewis-Säuren ausüben. Bisher wurden Lewis-Säuren (insbesondere Auminiumchlorid) nur in der homogenen Katalyse eingesetzt. Es ist daher überraschend, daß auch die austauschfähig gebundenen Al³⁺-Ionen in den erfindungsgemäßen heterogenen Katalysatoren nach Art von Lewis-Säuren wirken. Besonders überraschend ist, daß der katalytische Effekt der austauschfähigen Al³⁺-Kationen stärker ist als der katalytische Effekt der freien Al³⁺-Ionen.

Die erfindungsgemäß verwendeten Katalysatoren liegen vorzugsweise als Teilchen mit einer Größe von mehr als 0,1 mm vor, die nachstehend als "Granulate" bezeichnet werden. Darunter fallen auch Formkörper, wie Extrudate, Kugeln, Pellets usw. Diese Formköper können im allgemeinen bis zu etwa 10 mm groß sein.

Gegenstand der Erfindung ist ferner die Verwendung eines Katalysators, der dadurch hergestellt wird, daß man ein säureaktiviertes smektitisches Ausgangsmaterial mit einer Aluminiumsalzlösung behandelt, deren Menge so bemessen ist, daß sich der Gehalt an austauschfähigen Al³⁺-Ionen auf mindestens 10 mVal/100 g erhöht und daß gegebenenfalls noch ein Anteil an freien Al³⁺-Ionen vorliegt.

Hierbei geht man im allgemeinen von einem säureaktivierten smektitischen Ausgangsmaterial mit einem Gehalt an austauschfähigen Al³⁺-Ionen von weniger als 5mVal/100 g aus, obgleich man auch von einem Material mit einem höheren Gehalt an austauschfähigen Al³⁺-Ionen ausgehen kann. Die katalytische Aktivität eines derartigen Ausgangsmaterials, das bereits die Bedingungen für einen erfindungsgemäßen Katalysator erfüllt, kann durch den Eintausch von weiteren Al³⁺-Ionen noch gesteigert werden. Die erfindungsgemäße Behandlung kann also in zwei oder mehreren Stufen durchgeführt werden.

Bei der ersten bevorzugten Variante des erfindungsgemäßen Verfahrens geht man so vor, daß man das Aluminiumsalz in einem 1-bis 5-fachen molaren Überschuß verwendet, einen Eintausch der Al³⁺-Ionen gegen ein- und zweiwertige Kationen vornimmt und den Katalysator anschließend wäscht, um freie, d.h. wasserlösliche Aluminiumsalze sowie Salze der ein- und zweiwertigen Kationen zu entfernen, falls diese im Gesamtprozeß stören.

Hierbei wird eine Suspension des smektitischen Tonminerals (Bleicherdesuspension) mit einer Aluminiumsalzlösung, insbesondere mit einer Aluminiumsufatlösung, imprägniert, wobei ein direkter Zonenaustausch stattfindet. Nach mehrstündigem Austausch bei Raumtemperatur oder erhöhter Temperatur wird der Überschuß an Aluminiumionen ausgewaschen, worauf das ausgetauschte Bleicherdeprodukt anschließend getrocknet wird. Hieran schließt sich der Granulationsprozeß an.

Nach der zweiten Variante kann man ein zuvor klassiertes Granulat eines säureaktivierten Bentonits (Bleicherdegranulat) mit überschüssiger wäßriger Aluminiumsulfatlösung einsprühen, wobei ein Teil der Al³⁺-Ionen austauschfähig gebunden wird und ein anderer Teil der Al³⁺-Ionen in freier Form, d.h. lediglich adsorbiert vorliegt. Nach dem Einsprühen wird das Material bei etwa 80 bis 150°C, vorzugsweise bei etwa 100°C getrocknet.

Für eine erheblich gesteigerte katalytische Aktivität sind bei beiden Varianten einige Prozent Aluminiumsalz ausreichend. Grundsätzlich sind verschiedene Aluminiumsalze geeignet. Aus kommerziellen und technischen Gründen ist Aluminiumsulfat dem Aluminuimchlorid vorzuziehen.

Nach beiden Varianten kann man den mit Al³⁺-Ionen angereicherten Katalysator in ein Granulat mit einer Teilchengröße von > 0,1 mm überführen.

Die in den Ansprüchen und in der Beschreibung angebenen weiteren Merkmale werden wir folgt bestimmt:
1. Spezifische Oberfläche: nach der BET-Methode (Einpunktmethode mit Stickstoff nach DIN 66131, wobei die Probe zuvor 10 Stunden bei 150°C entgast wird).
2. Gesamtacidität Eine Probe des Katalysators (5 g) wird in 250 ml 5 Gew.-%iger NaCl-Lösung bei 95°C suspendiert und anschließend filtriert; das erhaltene klare Filtrat wird mit 0,1 n KOH-Lösung gegen Phenolphthalein titriert. Die Gesamtacidität wird in mg KOH/100 g Katalysator ausgedrückt.
3. Freie Kieselsäure: Bei der Säureaktivierung von smektitischen Tonmineralen werden zunächst die austauschfähigen Kationen auf den Zwischengitterplätzen durch Protonen ersetzt, worauf die in der Oktaederschicht gebundenen Kationen an den Rändern der Schichtpakete herausgelöst werden und amorphe Kieselsäure hinterbleibt. Der Anteil dieser amorphen (freien) Kieselsäure nimmt mit steigender Intensität der Säureaktivierung (Konzentration, Temperatur, Druck, Zeit) zu. Die freie Kieselsäure kann dadurch bestimmt werden, daß eine Probe (1 g) des säurekativierten Bentonits 10 min in 100 ml 2%-iger Sodalösung ausgekocht wird. Aus der freien Kieselsäure bildet sich lösliches Natriumsilicat, während das im Gitter gebundene Silicium nicht herausgelöst wird. Die in Lösung gegangene freie Kieselsäure kann dann in beliebiger Weise nach Filtration, z.B. nach der Natriummolybdat-Methode bestimmt werden.

Die Erfindung ist durch die nachstehenden Beispiel in nicht einschränkender Weise erläutert.

### Vergleichsbeispiel 1

### Herstellung eines Granulats aus unbehandetem Bentontit

100 kg eines getrockneten bayerischen Calciumbentonit (Tonsil® 13 der Fa. Süd-Chemie AG) mit den in Tabelle I angegebenen Eigenschaften werden gemahlen und im Drais-Intensivmischer mit 32 kg Wasser 10 Minuten gemischt und anschließend auf einem Einschneckenextruder zu 2 mm dicken Strängen verpreßt.

Die feuchten Stränge werden bei 110° C 10 Stunden getrocknet und anschließend in einem Walzenbrecher mit einem Spaltabstand von 1 mm zerkleinert. Anschließend wird die Fraktion zwischen 0,3 mm und 0,6 mm abgesiebt.

### Vergleichsbeispiel 2

### Herstellung eines unmodifizierten Bleicherdearanulats

100 kg einer Bleicherde in Form eines salzsauer aktivierten Bentonits (Tonsil® Optimum FF der Fa. Süd-Chemie AG) mit den in Tabelle 1 angegebenen Eigenschaften werden im Drais-Intensivmischer mit 40 kg Wasser 10 Minuten gemischt und anschließend auf einem Einschneckenextruder zu 2 mm dicken Strängen verpreßt.

Die feuchten Stränge werden wie nach Vergleichsbeispiel 1 weiterbehandelt.

### Beispiel 1

### Imprägnierung von säureaktiviertem Bentonit mit Aluminiumsulfat

100 kg des Bleicherdegranulats von Vergleichsbeispiel 2 werden in einen Pelletisierteller gebracht und anschließend mit 20 Liter einer 25 %igen Aluminiumsulfatlösung besprüht. Das imprägnierte Granulat wird 24 Stunden bei 120 °C getrocknet.

Die Al³⁺-Ionen sind zum Teil in das Gitter eingetauscht; zum Teil befinden sie sich auf der Oberfläche des Materials. Die Eigenschaften des imprägnierten Granulats sind in Tabelle I angegeben.

### Beispiel 2

### (Eintausch von Al³⁺-Ionen in säureaktivierten Bentonit

100 kg des säureaktivierten Bentonits von Vergleichsbeipiel 2, werden in 500 Liter destilliertem Wasser unter starkem Rühren dispergiert. Nach einer Rührzeit von 3 Stunden werden 20 kg Aluminiumsulfat in fester Form zugegeben und anschliessend bei Raumtemperatur weitere 12 Stunden gerührt.

Die aluminiumsulfat-haltige Suspension wird anschließend auf einer Filterpresse entwässert und mit 1000 Liter demineralisiertem Wasser nachgewaschen. Der feuchte Filterkuchen wird 12 Stunden bei 120° C getrocknet.

Dann wird der getrocknete Filterkuchen auf einem Brecher zerkleinert und die Fraktion zwischen 0,3 und 0,6 mm abgesiebt. Die Eigenschaften des so erhaltenen Katalysators sind in Tabelle I angegeben.

### Anwendungsbeispiel

### (Bestimmung der katalytischen Aktivität der Katalysatoren bei der Olefinentfernung aus Aromatengemischen)

Ein aus der Sulfolanextraktion erhaltenes Aromatengemisch (70 Gew.-% Benzol, 30 Gew.-% Toluol) mit einem Olefinanteil entsprechen einem Bromindex von 50 mg Br₂/100 g (nach ASTM D1491) wird mit einer HPLC-Pumpe über eine HPLC-Säule geleitet, in der sich der zu untersuchende Katalysator als Schüttung befindet (Reaktorvolumen 10 ml). Die HPLC-Säule befindet sich in einem regelbaren Thermostat, in dem die Temperatur bei 200° C konstant gehalten wird. Um die Gasbildung bei diesen hohen Temperaturen zu vermeiden, befindet sich zwischen HPLC-Säule und den elektronisch gesteuerten Probenahmenventilen ein Rückdruckregler. An diesem Rückdruckregler wird ein Gegendruck von 50 bar eingestellt.

Mit Hilfe der HPLC-Pumpe wird eine LHSV (liquid hourly space velocity = Flüssig-Raumgeschwindigkeit) von 12 h⁻¹ eingestellt. Über die zeitgesteuerten Probenahmeventile wird alle 24 Stunden eine Probe der gereinigten Aromatenmischung entnommen und der Bromindex bestimmt.

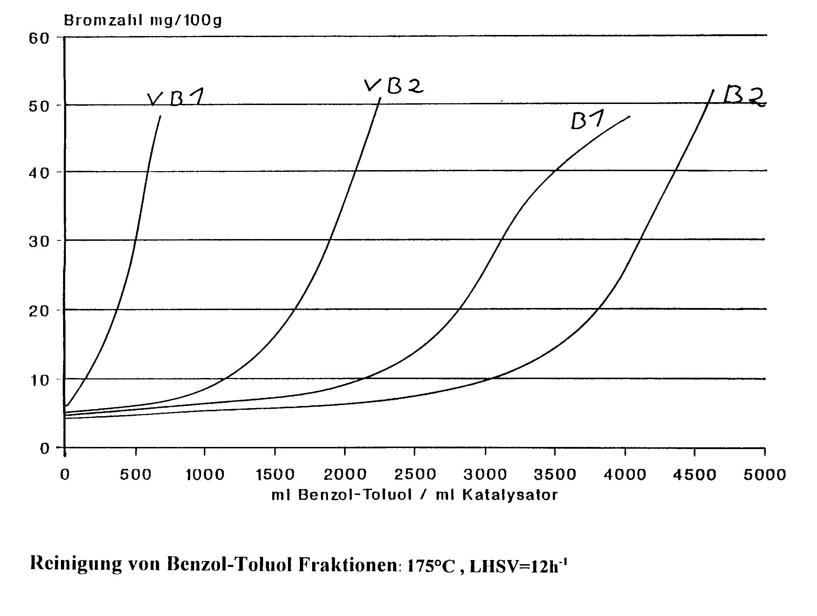

Zur Beurteilung der katalytischen Aktivität wird der Bromindex gegen die Betriebsdauer (Tage) aufgetragen. Die Ergebnisse sind in der beigefügten Figur angegeben.

Man erkennt, daß der säureaktivierte Bentonit nach Vergleichsbeispiel 2 eine längere Standzeit als der unbehandelte Bentonit nach Vergleichsbeispiel 1. Die längste Standzeit hat je doch der Katalysator nach Beispiel 2, der nur austauschfähige Al³⁺-Kationen enthält. Eine etwas geringere Standzeit hat der Katalysator nach Beispiel 1, der neben den austauschbaren Al³⁺-Ionen auch freie Al³⁺-Ionen enhält. Daraus ergibt sich, daß der erfindungsgemäße Effekt dann am ausgeprägtesten ist, wenn die Al³⁺-Ionen auf den Zwischenschichtplätzen des säureaktivierten Bentonits austauschfähig gebunden sind.

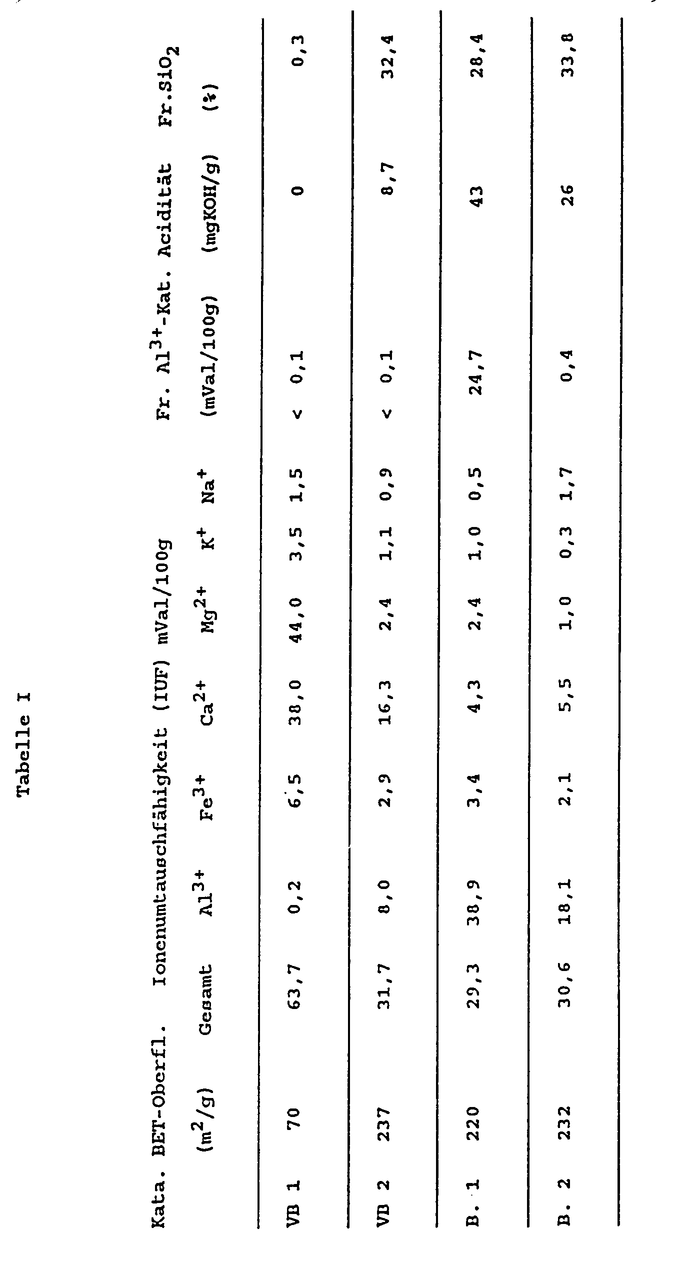

## Patentansprüche

1. Verwendung eines Katalysators auf der Basis eines säureaktivierten smektitischen Tonminerals, welches mindestens 5 mVal/100g austauschfähige Al³⁺-Kationen enthält, zur Entfernung von Olefinen aus Aromaten oder Aromatengemischen.

2. Verwendung nach Anspruch 1, dadurch gekennzeichnet, daß das säureaktivierte smektitische Tonmineral die nachstehend angegebenen Eigenschaften hat:
(a) eine spezifische Oberfläche von etwa 50 bis 500 m²/g;
(b) eine Ionenumtauschfähigkeit (IUF) für austauschfähige Kationen von insgesamt etwa 20 bis 60 mVal/100 g, wobei der Anteil der Al³⁺-Ionen an der IUF mindestens 5 mVal/100g, vorzugsweise etwa 10 bis 60 mVal/100 g, der Anteil der Alkali-Ionen an der IUF weniger als etwa 7,0 mVal/100 g und der Anteil der Erdalkali-Ionen an der IUF weniger als etwa 50 mVal/100 g beträgt;
(c) eine Gesamtacidität von mehr als etwa 15 mg KOH/g;
(d) einen Anteil an freien Erdalkali- oder Alkali-Ionen von weniger als 5 mVal/100g;
(e) einen Gehalt an freier Kieselsäure von etwa 15 bis 55 Gew.-%.

3. Verwendung nach Anspruch 1 oder 2, dadurch gekennzeichnet, daß der Gehalt des Katalysators an austauschfähigen Al³⁺-Ionen etwa 10 bis 50 mVal/100 g beträgt.

4. Verwendung nach einem der Ansprüche 1 bis 3, dadurch gekennzeichnet, daß der Katalysator zusätzlich zu den austauschfähigen Al³⁺-Ionen freie Al³⁺-Ionen auf der Oberfläche, in den Poren und im Zwischenkornvolumen enthält.

5. Verwendung nach einem der Ansprüche 1 bis 4, dadurch gekennzeichnet, daß der Katalysator in Form von Teilchen von mehr als etwa 0,1 mm vorliegt.

6. Verwendung nach einem der Ansprüche 1 bis 5, dadurch gekennzeichnet, daß der Katalysator dadurch hergestellt wird, daß man ein säureaktiviertes smektitisches Ausgangsmaterial mit einer Aluminiumsalzlösung behandelt, deren Menge so bemessen ist, daß sich der Gehalt an austauschfähigen Al³⁺-Ionen auf mindestens 5 mVal/100 g erhöht und daß gegebenenfalls noch ein Anteil an freien Al³⁺-Ionen vorliegt.

7. Verwendung nach Anspruh 2, 3 oder 4, dadurch gekennzeichnet, daß der Katalysator dadurch hergestellt wird, daß man das Aluminiumsalz in einem 1- bis 5-fachen molaren Überschuß, bezogen auf die IUF verwendet, einen Eintausch der Al³⁺-Ionen gegen ein- und zweiwertige Kationen vornimmt und den Katalysator anschließend wäscht.

8. Verwendung nach Anspruch 6 oder 7, dadurch gekennzeichnet, daß man den mit Al³⁺-Ionen angereicherten Katalysator in Formkörper mit einer Teilchengröße von > 0,1 mm überführt.

## Claims

1. Use of a catalyst based on an acid-activated smectitic clay mineral, which contains at least 5 meq/100g of exchangeable Al³⁺ cations, for removing olefins from aromatic compounds or aromatic mixtures.

2. Use according to Claim 1, characterised in that the acid-activated smectitic clay mineral has the properties indicated below:
(a) a specific surface area of approximately 50 to 500m²/g;
(b) an ion exchange capacity (IEC) for exchangeable cations of, in total, approximately 20 to 60 meq/100g, wherein the proportion of Al³⁺ ions in terms of the IEC is at least 5 meq/100g, preferably approximately 10 to 60 meq/100g, the proportion of alkali ions in terms of the IEC is less than approximately 7.0 meq/100g and the proportion of alkaline earth ions in terms of the IEC is less than approximately 50 meq/100g;
(c) a total acidity of more than approximately 15 mg KOH/g;
(d) a proportion of free alkaline earth ions or alkali ions of less than 5 meq/100g;
(e) a content of free silicic acid of approximately 15 to 55% by weight.

3. Use according to Claim 1 or 2, characterised in that the content in the catalyst of exchangeable Al³⁺ ions is approximately 10 to 50 meq/100g.

4. Use according to any one of Claims 1 to 3, characterised in that, in addition to the exchangeable Al³⁺ ions, the catalyst contains free Al³⁺ ions on the surface, in the pores and in the intergranular volume.

5. Use according to any one of Claims 1 to 4, characterised in that the catalyst is present in the form of particles of more than approximately 0.1 mm.

6. Use according to any one of Claims 1 to 5, characterised in that the catalyst is prepared by treating an acid-activated smectitic starting material with an aluminium salt solution, the quantity of which is calculated so that the content of exchangeable Al³⁺ ions is increased to at least 5 meq/100 g and that a proportion of free Al³⁺ ions is optionally also present.

7. Use according to Claim 2, 3 or 4 characterised in that the catalyst is prepared by using the aluminium salt in a 1 to 5-fold molar excess, based on the IEC, by effecting an exchange of the Al³⁺ ions for monovalent and divalent cations and by subsequently washing the catalyst.

8. Use according to Claim 6 or 7, characterised in that catalyst, which has been enriched with Al³⁺ ions, is transformed into moulded articles with a particle size of > 0.1 mm.

## Revendications

1. Utilisation d'un catalyseur à base d'un minéral argileux de type smectite activé par un acide, qui contient au moins 5 mVal/100 g de cations Al³⁺ échangeables, pour éliminer les oléfines à partir de composés aromatiques ou de mélanges de composés aromatiques.

2. Utilisation selon la revendication 1, caractérisée en ce que le minéral argileux de type smectite activé par un acide a les propriétés suivantes :
(a) une aire spécifique d'environ 50 à 500 m²/g ;
(b) une capacité totale d'échange d'ions (CEI) pour les cations échangeables, d'environ 20 à 60 mVal/100 g, la contribution des ions Al³⁺ à la CEI étant d'au moins 5 mVal/100 g et de préférence d'environ 10 à 60 mVal/100 g, la contribution des ions alcalins à la CEI étant inférieure à environ 7,0 mval/100 g, et la contribution des ions de métaux alcalino-terreux à la CEI étant inférieure à environ 50 mVal/100 g ;
(c) une acidité totale supérieure à environ 15 mg KOH/g ;
(d) une proportion d'ions libres de métaux alcalins ou alcalino-terreux inférieure à 5 mval/100 g ;
(e) une teneur en silice libre d'environ 15 à 55 % en poids.

3. Utilisation selon la revendication 1 ou 2, caractérisée en ce que la teneur du catalyseur en ions Al³⁺ échangeables est d'environ 10 à 50 mVal/100 g.

4. Utilisation selon l'une des revendications 1 à 3, caractérisée en ce que le catalyseur contient, outre les ions Al³⁺ échangeables, des ions Al³⁺ sur la surface, dans les pores et dans les volumes intergranulaires.

5. Utilisation selon l'une des revendications 1 à 4, caractérisée en ce que le catalyseur se présente sous forme de particules ayant une granulométrie supérieure à environ 0,1 mm.

6. Utilisation selon l'une des revendications 1 à 5, caractérisée en ce qu'on prépare le catalyseur en traitant une matière de départ de type smectite et activée par un acide avec une solution d'un sel d'aluminium dont la quantité est définie de façon que la teneur en ions Al³⁺ échangeables augmente à au moins 5 mVal/100 g, et que l'on soit encore éventuellement en présence d'une certaine proportion d'ions Al³⁺ libres.

7. Utilisation selon la revendication 2, 3 ou 4, caractérisée en ce qu'on prépare le catalyseur en utilisant le sel d'aluminium selon un excès molaire 1x à 5x par rapport à la CEI, on procède à un échange des ions Al³⁺ par des cations monovalents et divalents, puis on lave le catalyseur.

8. Utilisation selon la revendication 6 ou 7, caractérisée en ce qu'on transforme le catalyseur, enrichi en ions Al³⁺, en des objets façonnés ayant une granulométrie supérieure à 0,1 mm.
